# EUROPEAN PATENT APPLICATION

(11) **EP 4 790 419 A1**
(43) Date of publication of application: **12.08.2026**
(21) Application number: 24874667.9
(22) Date of filing: 03.10.2024
(51) Int. Cl.: G01N 33/68, G01N 27/62, G01N 33/574

(54) **METHOD FOR DETECTING CANCER OF DIGESTIVE ORGAN**

(30) Priority: 04.10.2023 JP 2023172685
(71) Applicant: Tohoku University, Sendai-shi, Miyagi 980-8577 (JP)
(72) Inventor: ASAMITSU Seji, Sendai-shi, Miyagi 980-8577 (JP); AKAIKE Takaaki, Sendai-shi, Miyagi 980-8577 (JP); OZAWA Yohei, Sendai-shi, Miyagi 980-8577 (JP); OKAMOTO Hiroshi, Sendai-shi, Miyagi 980-8577 (JP); KAMEI Takashi, Sendai-shi, Miyagi 980-8577 (JP); MOTOHASHI Hozumi, Sendai-shi, Miyagi 980-8577 (JP)
(74) Representative: Hoffmann Eitle
(86) International application number: PCT/JP2024/035368
(87) International publication number: WO 2025/075067

(57) **Abstract**

The invention provides a method for detecting a cancer of a digestive organ in a subject, the method including a step of measuring an amount of cysteine persulfide or homocysteine persulfide in a sample derived from the subject. The sample is a sample derived from the subject other than a digestive organ tissue suspected of having a cancer. The invention also provides a method for diagnosing a cancer of a digestive organ and a method for treating a digestive organ, using the above method.

## Description

### Technical Field

### Related Application:

The present description incorporates the contents described in the description of Japanese Patent Application No. 2023-172685 (filed on October 4, 2023), which is the basis of priority for the present application.

### Technical field:

The present invention relates to a method for detecting a cancer of a digestive organ. More specifically, the invention relates to a method for detecting a cancer of a digestive organ using, as an indicator, an amount of cysteine persulfide or homocysteine persulfide in a sample derived from a subject other than a digestive organ tissue suspected of having a cancer.

### Background Art

In recent years, incidence of a cancer of a digestive organ such as gastric cancer, colorectal cancer, and esophageal cancer tends to increase with changes in dietary habits. The cancer of the digestive organ has almost no subjective symptoms at an early stage, which causes difficulty in treatment of the cancer of the digestive organ.

Currently, screening for medical examination of the cancer of the digestive organ is widely performed by barium fluoroscopy, but detection accuracy thereof is low, and it is difficult to identify an abnormality unless apparent deformation occurs in a considerably advanced cancer. Therefore, importance of medical examination screening by endoscopic examination using gastroscopy, colonoscopy, or the like is recognized, but such screening is not sufficiently widespread since examination cost including equipment is high, the examination is invasive, and thus causes discomfort to an examinee.

As tumor markers used clinically, there are squamous cell carcinoma antigen (SCC), carcinoembryonic antigen (CEA), CA19-9, and the like, which are useful for monitoring recurrence and the like, but these are not suitable for screening since utility in early diagnosis has not been established.

As a biomarker for early and non-invasive detection of the cancer of the digestive organ, detection of a cancer by a biomarker in a blood sample or an exhaled breath sample has also been studied. In particular, unlike blood and tissue samples, the exhaled breath sample can be collected in a non-invasive manner, but research on a biomarker for the cancer of the digestive organ in exhaled breath has not progressed.

In vivo, a large amount of supersulfur molecules such as cysteine persulfide are synthesized and exert diverse physiological functions. The inventors have previously reported that a sulfur metabolite can be used as a biomarker for an infectious disease (PTL 1) and that a supersulfur metabolite is decreased in lung cells and airway epithelial lining fluid obtained from a patient with chronic obstructive pulmonary disease and related inflammatory airway disease (NPLs 1 and 2). It has also been reported that a level of sulfur-related substances is higher in a colorectal cancer tissue than in a normal tissue (NPL 3). However, it is not clarified how an amount of a sulfur metabolite in a sample other than a cancer tissue, such as blood or exhaled breath, and which sulfur metabolite reflect cancer lesions.

### Citation List

### Patent Literature

PTL 1: JP2022-178150A

### Non Patent Literature

NPL 1: Numakura et al. (2017) Production of reactive persulfide species in chronic obstructive pulmonary disease. Thorax, 72, pp1074-1083
NPL 2: Kyogoku et al. (2019) Nitrosative stress in patients with asthma-chronic obstructive pulmonary disease overlap. J. Allergy Clin. Immunol., 144, pp972-983.e14.
NPL 3: Fukuoka et al. (2021) Sulphur metabolism in colon cancer tissues: a case report and literature review. J. Int. Med. Res., (2021), 49(11), pp1-11

### Summary of Invention

### Technical Problem

An object of the invention is to specify a new biomarker for early diagnosis of a cancer of a digestive organ and to provide a method for diagnosing a cancer of a digestive organ using the biomarker early in a non-invasive manner.

### Solution to Problem

The present inventors performed comprehensive quantitative measurements of sulfur metabolites in exhaled breath condensate and plasma samples from esophageal cancer patients and healthy subjects. As a result, the present inventors have found that cysteine persulfide and homocysteine persulfide are significantly high in the esophageal cancer patients and can be new biomarkers for detecting a cancer of a digestive organ such as esophageal cancer.

The invention is based on the above findings, and provides the following detection methods [1] to [11] in a first aspect.
[1] A method for detecting a cancer of a digestive organ in a subject, the method including:
   a step of measuring an amount of cysteine persulfide or homocysteine persulfide in a sample derived from the subject, in which
   the sample is a sample derived from the subject other than a digestive organ tissue suspected of having a cancer.
[2] The method according to [1], in which
   the sample derived from the subject is a sample obtained from exhaled breath or blood of the subject.
[3] The method according to [1] or [2], in which
   the sample derived from the subject is an exhaled breath condensate or plasma of the subject.
[4] The method according to any one of [1] to [3], in which
   the cancer of the digestive organ is squamous cell carcinoma or adenocarcinoma.
[5] The method according to any one of [1] to [4], in which
   the cancer of the digestive organ is esophageal cancer, colorectal cancer, or pancreatic cancer.
[6] The method according to any one of [1] to [5], in which
   the amount of the cysteine persulfide or the homocysteine persulfide being equal to or greater than a predetermined cutoff value indicates a possibility that the subject has the cancer of the digestive organ.
[7] The method according to any one of [1] to [6], in which
   the amount of the cysteine persulfide or the homocysteine persulfide is measured using a mass spectrometer.
[8] The method according to any one of [1] to [7], in which
   the amount of the cysteine persulfide or the homocysteine persulfide is measured by a stable isotope dilution method.
[9] The method according to any one of [1] to [8], further including:
   a step of reacting an electrophilic alkylating agent having a hydroxyphenyl group with the sample derived from the subject.
[10] The method according to [9], in which
   the electrophilic alkylating agent having the hydroxyphenyl group is β-(4-hydroxyphenyl)ethyl iodoacetamide (HPE-IAM).
[11] The method according to [10], in which
   the HPE-IAM is labeled with a stable isotope.

In a second aspect, the invention provides the following diagnostic methods [1] to [11].
[1] A method for diagnosing a cancer of a digestive organ, the method including:
   a step of measuring an amount of cysteine persulfide or homocysteine persulfide in a sample derived from a subject, in which
   the sample is a sample derived from the subject other than a digestive organ tissue suspected of having a cancer.
[2] The method according to [1], in which
   the sample derived from the subject is a sample obtained from exhaled breath or blood of the subject.
[3] The method according to [1] or [2], in which
   the sample derived from the subject is an exhaled breath condensate or plasma of the subject.
[4] The method according to any one of [1] to [3], in which
   the cancer of the digestive organ is squamous cell carcinoma or adenocarcinoma.
[5] The method according to any one of [1] to [4], in which
   the cancer of the digestive organ is esophageal cancer, colorectal cancer, or pancreatic cancer.
[6] The method according to any one of [1] to [5], in which
   the amount of the cysteine persulfide or the homocysteine persulfide being equal to or greater than a predetermined cutoff value indicates a possibility that the subject has the cancer of the digestive organ.
[7] The method according to any one of [1] to [6], in which
   the amount of the cysteine persulfide or the homocysteine persulfide is measured using a mass spectrometer.
[8] The method according to any one of [1] to [7], in which
   the amount of the cysteine persulfide or the homocysteine persulfide is measured by a stable isotope dilution method.
[9] The method according to any one of [1] to [8], further including:
   a step of reacting an electrophilic alkylating agent having a hydroxyphenyl group with the sample derived from the subject.
[10] The method according to [9], in which
   the electrophilic alkylating agent having the hydroxyphenyl group is β-(4-hydroxyphenyl)ethyl iodoacetamide (HPE-IAM).
[11] The method according to [10], in which
   the HPE-IAM is labeled with a stable isotope.

In a third aspect, the invention also provides the following treatment method [12].
[12] A method for treating a cancer of a digestive organ, including: a step of diagnosing, by the diagnostic method according to [1] to [11] above, whether the subject has the cancer of the digestive organ; and a step of performing the following treatment on the subject diagnosed as having the cancer of the digestive organ, in which the sample is a sample derived from the subject other than a digestive organ tissue suspected of having a cancer.
(A) When the cancer of the digestive organ is esophageal squamous cell carcinoma, any treatment selected from the following i) to vii):
   i) administration of one or two or more anticancer agents selected from cisplatin, fluorouracil, docetaxel, and paclitaxel;
   ii) administration of docetaxel, cisplatin and fluorouracil;
   iii) administration of cisplatin and fluorouracil;
   iv) therapy of a combination of fluorouracil, levofolinate, and oxaliplatin (FOLFOX);
   v) administration of an immune checkpoint inhibitor such as pembrolizumab, nivolumab, or ipilimumab;
   vi) a combination of any one of the above i) to iv) with an immune checkpoint inhibitor such as pembrolizumab, nivolumab, or ipilimumab; and
   vii) a combination of carboplatin, paclitaxel, and radiation therapy.
(B) When the cancer of the digestive organ is esophageal or gastric adenocarcinoma, any treatment selected from the following i) to v):
   i) a combination of oxaliplatin and S-1 (SOX therapy);
   ii) a combination of docetaxel, oxaliplatin, 5-FU, and leucovorin (FLOT therapy);
   iii) therapy of a combination of fluorouracil, levofolinate, and oxaliplatin (FOLFOX);
   iv) a combination of any one of the above i) to iii) and trastuzumab for HER2-positive esophageal adenocarcinoma; and
   v) a combination of carboplatin, paclitaxel, and radiation therapy.

### Advantageous Effects of Invention

The invention enables early and non-invasive detection and diagnosis of a cancer of a digestive organ, and contributes to treatment thereof.

### Brief Description of Drawings

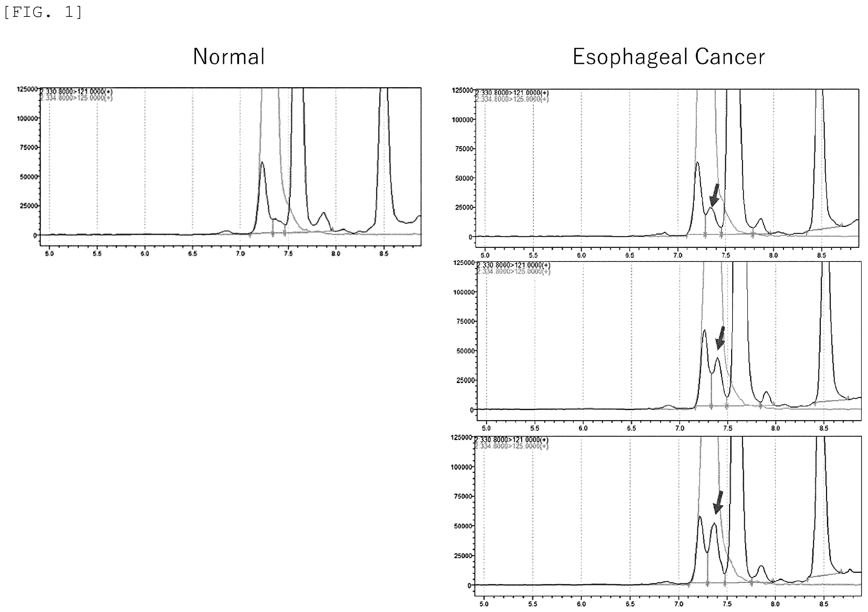

[FIG. 1] FIG. 1 shows peaks of CysSS-HPE-IAM in exhaled breath condensates of esophageal cancer patients and healthy subjects. Peaks of CysSS are observed only for the esophageal cancer patients.

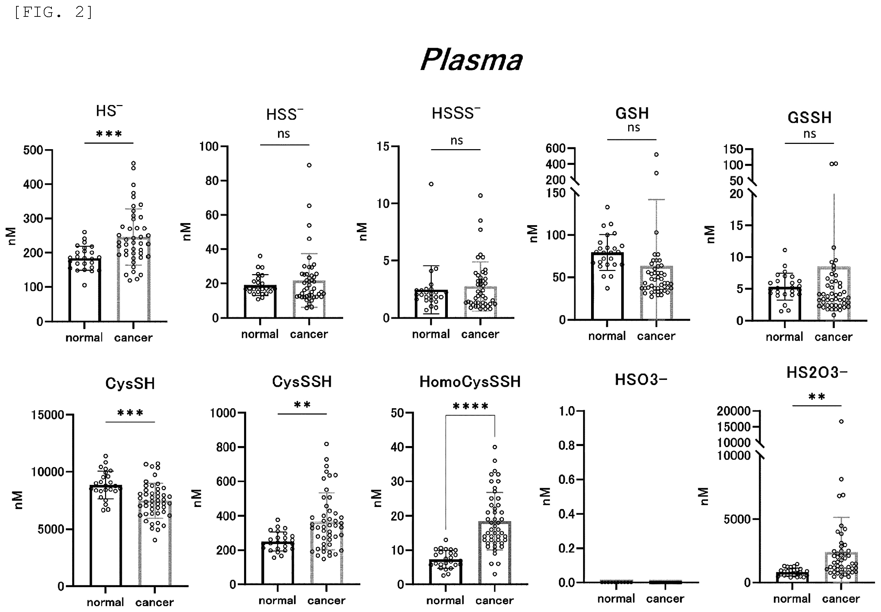

[FIG. 2] FIG. 2 shows a concentration of each sulfur metabolite (HS-, HSS-, HSSS-, GSH, GSSH, CysSH, CysSSH, HomoCysSSH, HSO₃⁻, HS₂O₃⁻) in plasma samples. Normal (healthy subject: n = 25), Cancer (esophageal cancer patient: n = 47); *: p < 0.05, **: p < 0.01, ***: p < 0.001, ****: p < 0.0001

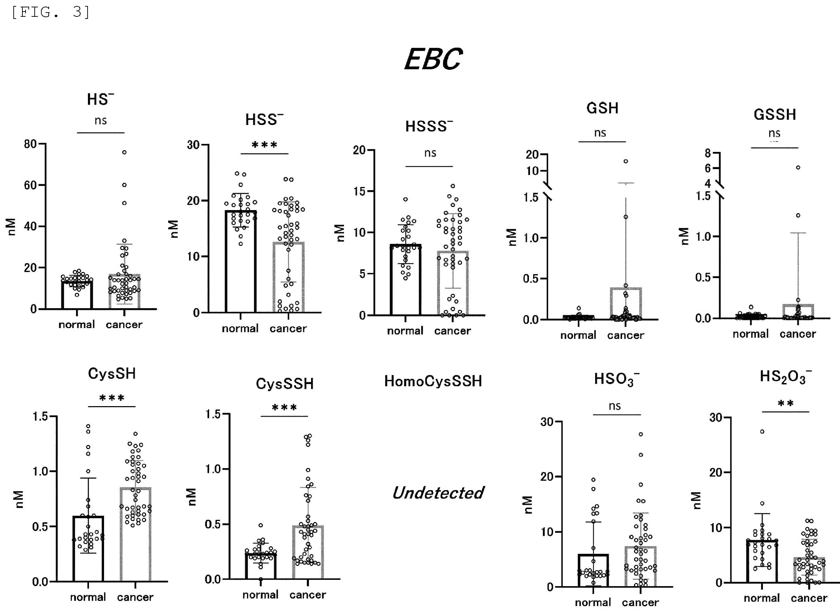

[FIG. 3] FIG. 3 shows a concentration of each sulfur metabolite (HS-, HSS-, HSSS-, GSH, GSSH, CysSH, CysSSH, HomoCysSSH, HSO₃⁻, HS₂O₃⁻) in an exhaled breath condensate. Normal (healthy subject: n = 26), Cancer (esophageal cancer patient: n = 44); *: p < 0.05, **: p < 0.01, ***: p < 0.001, ****: p < 0.0001

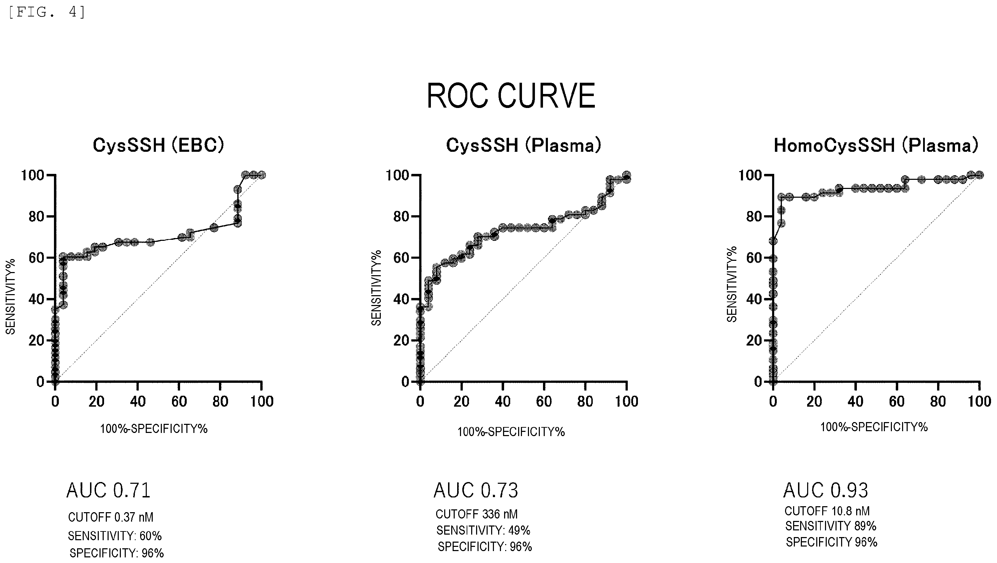

[FIG. 4] FIG. 4 shows ROC curves for cysteine persulfide (CysSSH) and homocysteine persulfide (HomoCysSSH). From the left, CysSSH in an exhaled breath condensate (EBC) (AUC 0.71: at a cutoff of 0.37 nM, sensitivity 60%, specificity 96%), CysSSH in plasma (AUC 0.73: at a cutoff of 336 nM, sensitivity 49%, specificity 96%), and HomoCysSSH in plasma (AUC 0.93: at a cutoff of 10.8 nM, sensitivity 89%, specificity 96%).

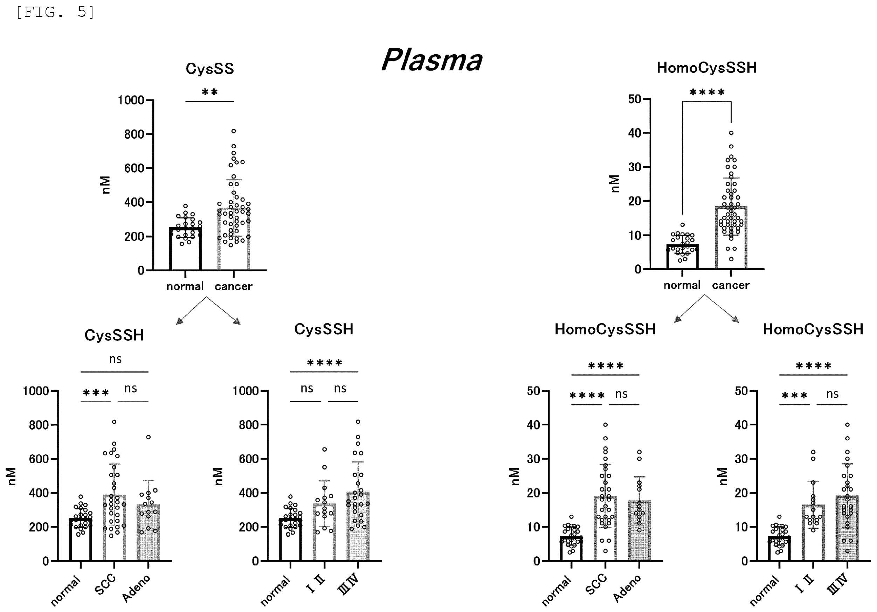

[FIG. 5] FIG. 5 shows results of comparing concentrations of cysteine persulfide (CysSSH) and homocysteine persulfide (HomoCysSSH) in plasma between esophageal cancer patients and healthy subjects, where the patients are classified into squamous cell carcinoma (SCC) and adenocarcinoma (Adeno), and into Stage I-II and Stage III-IV. Normal (healthy subject: n = 25), Cancer (esophageal cancer patient: n = 47); *: p < 0.05, **: p < 0.01, ***: p < 0.001, ****: p < 0.0001

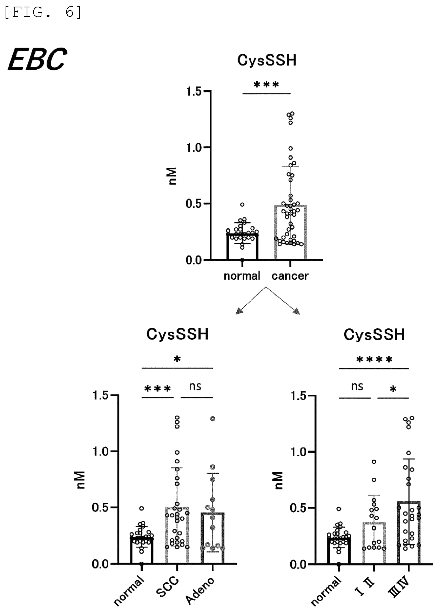

[FIG. 6] FIG. 6 shows results of comparing concentrations of cysteine persulfide (CysSSH) in exhaled breath condensates between esophageal cancer patients and healthy subjects, where the patients are classified into squamous cell carcinoma (SCC) and adenocarcinoma (Adeno), and into Stage I-II and Stage III-IV. Normal (healthy subject: n = 26), Cancer (esophageal cancer patient: n = 44); *: p < 0.05, **: p < 0.01, ***: p < 0.001, ****: p < 0.0001

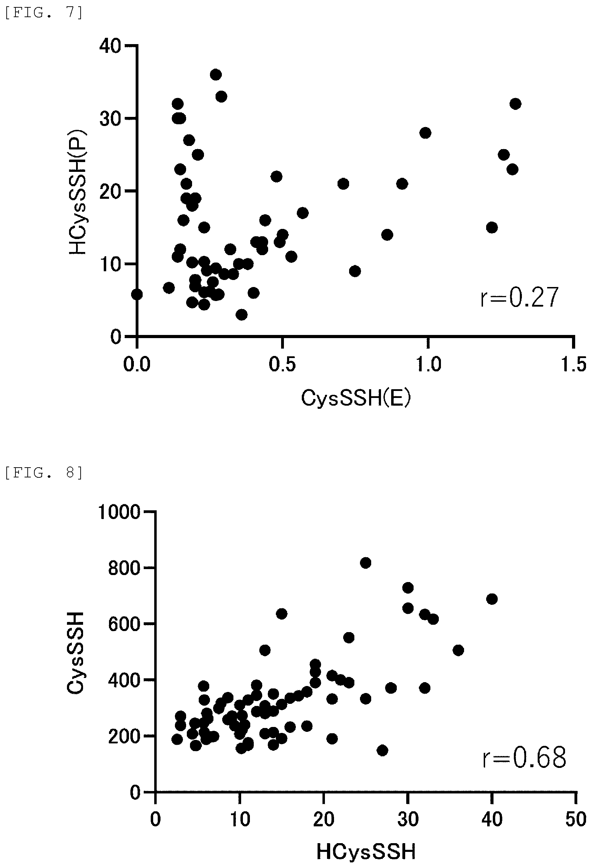

[FIG. 7] FIG. 7 shows a correlation between a concentration of cysteine persulfide (CysSSH) in an exhaled breath condensate and a concentration of homocysteine persulfide (HomoCysSSH) in plasma. n = 72 (Normal (healthy subject: n = 25), Cancer (esophageal cancer patient: n = 47)), correlation coefficient r = 0.27
[FIG. 8] FIG. 8 shows a correlation between the concentration of homocysteine persulfide (HomoCysSSH) in plasma and a concentration of cysteine persulfide (CysSSH) in plasma. n = 72 (Normal (healthy subject: n = 25), Cancer (esophageal cancer patient: n = 47)), correlation coefficient r = 0.68

### Description of Embodiments

### 1. Method for Detecting Cancer of Digestive Organ

The invention relates to a method for detecting a cancer of a digestive organ in a subject using, as an indicator, an amount (concentration) of cysteine persulfide or homocysteine persulfide in a sample derived from the subject.

In the description, a "cancer of a digestive organ" is not particularly limited as long as the cancer occurs in a digestive organ. The digestive organ means an organ responsible for ingestion, absorption, digestion, and transport of ingested food, absorption of digested food, and excretion, and includes, for example, oral cavity, pharynx, esophagus, stomach, small intestine, large intestine, liver, gallbladder, pancreas, peritoneum, and a part or appendages thereof. Examples of the "cancer of a digestive organ" include esophageal cancer, gastric cancer, colorectal cancer, small intestinal cancer, liver cancer, gallbladder cancer, pancreatic cancer, and bile duct cancer. Among such cancers of digestive organs, the invention is suitable for detection of esophageal cancer, colorectal cancer, pancreatic cancer, and bile duct cancer, particularly suitable for detection of esophageal cancer, colorectal cancer, and pancreatic cancer, and among these, the invention is particularly suitable for detection of esophageal cancer that has no useful biomarker.

In the description, the cancer of the digestive organ may be squamous cell carcinoma or adenocarcinoma. The term "squamous cell carcinoma" refers to a cancer occurring from squamous cells, and the term "adenocarcinoma" refers to a cancer occurring from glandular cells (glandular epithelial cells).

In the description, the cancer of the digestive organ may be at any stage of progression (clinical stage), and may be a cancer at clinical stage I-II or clinical stage III-IV that is more advanced.

In the description, a "subject" is a mammal, preferably a primate, and more preferably a human.

In the description, a "sample derived from a subject" is not particularly limited as long as the sample is a sample other than a digestive organ tissue suspected of having a cancer and is a sample that can be isolated from the subject, and examples thereof include blood, saliva, sputum, urine, and skin. Among these, as the sample derived from the subject, blood or exhaled breath is preferable, and exhaled breath is preferable from the viewpoint of being non-invasive.

The term "blood" may be any one of whole blood, serum, and plasma, and plasma is preferable. Collection of the blood from the subject can be performed according to a method known in the art. An amount of blood to be collected is 0.1 ml or more, preferably about 0.1 ml to 0.3 ml as a plasma component.

As for "exhaled breath", since both volatile and nonvolatile compounds are to be analyzed, it is preferable to use an exhaled breath condensate. The term "exhaled breath condensate" is a liquid obtained by cooling and condensing exhaled breath at a low temperature (for example, 0°C to - 20°C).

The exhaled breath can be collected through a mouthpiece or the like according to a known method. The exhaled breath condensate can be obtained by cooling the collected exhaled breath. The exhaled breath condensate may be collected using a commercially available exhaled breath condensate collecting device, for example, ECoScreen (registered trademark), Turbo DECCS system, or RTube^{TM}.

In the invention, the amount (concentration) of cysteine persulfide or homocysteine persulfide in the sample derived from the subject is measured.

The term "cysteine persulfide" is a molecule where an excess sulfur atom is added to a SH group of cysteine, and the term "homocysteine persulfide" is a molecule where an excess sulfur atom is added to a SH group of homocysteine. Cysteine persulfide and homocysteine persulfide are reactive sulfur molecules rich in nucleophilic reactivity since an excess sulfur atom is added to a thiol group.

Detection and quantification of cysteine persulfide or homocysteine persulfide can be performed using, for example, a mass spectrometer (for example, LC-MS/MS, Q-TOF) (Ida et al. (2014). Proc. Natl. Acad. Sci. USA, 111, 7606-7611).

Since reduced reactive sulfur molecules such as cysteine persulfide or homocysteine persulfide are unstable, direct detection is difficult. Therefore, it is preferable to alkylate a terminal SH group using an electrophilic alkylating agent to obtain a stable derivative. As the electrophilic alkylating agent, N-ethylmaleimide (NEM), iodoacetamide (IAM), monobromobimane (MBB), and the like are known, but the electrophilic alkylating agent may destabilize (decompose) reactive sulfur molecules and affect quantification.

The inventors have found that a hydroxy compound, particularly a compound having a hydroxyphenyl group, has an effect of stabilizing polysulfides (JP2021-66722). Among electrophilic alkylating agents, β-(4-hydroxyphenyl)ethyl iodoacetamide (HPE-IAM), which is an IAM derivative having a hydroxyphenyl group, has the effect of stabilizing polysulfides, and thus is preferable for quantification of reactive sulfur molecules. Therefore, a detection method of the invention preferably includes a step of reacting HPE-IAM with the sample derived from the subject.

Cysteine persulfide or homocysteine persulfide can be detected using an appropriate label. Examples of such a label include fluorescent substances (fluorescent probes) and isotopes (stable isotopes and radioactive isotopes). The label used in the invention is preferably a fluorescent substance or an isotope, and particularly preferably a stable isotope.

The quantification of cysteine persulfide or homocysteine persulfide can be accurately performed using an appropriate internal standard such as a stable isotope (stable isotope dilution method). Other sulfur-related substances are also present in the sample from the subject, and an isotope dilution method is suitable for quantifying a specific substance in a mixture containing substances having fairly similar chemical properties. For example, the electrophilic alkylating agent such as HPE-IAM described above is labeled with the stable isotope, the electrophilic alkylating agent labeled with the stable isotope is reacted with the sample derived from the subject, and a stable isotope-labeled internal standard is added thereto to perform quantification. FIG. 1 shows quantification of cysteine persulfide by LC-MS/MS using stable isotope-labeled HPE-IAM and a stable isotope internal standard (Example 1).

A possibility that the subject has a cancer of a digestive organ can be determined based on whether the amount of cysteine persulfide or homocysteine persulfide is equal to or greater than a predetermined cutoff value. The cutoff value is appropriately determined depending on a type of the cancer of the digestive organ, the reactive sulfur molecule (cysteine persulfide or homocysteine persulfide) to be measured in the sample, a detection and quantification method, and the like.

For example, in the method of Example 1 to be described later, when 0.37 nM is set as a cutoff value for CysSSH in an exhaled breath condensate (EBC), a cancer can be detected with a sensitivity of 60% and a specificity of 96%, when 336 nM is set as a cutoff value for CysSSH in plasma, a cancer can be detected with a sensitivity of 49% and a specificity of 96%, and when 0.93 nM is set as a cutoff value for HomoCysSSH in plasma, a cancer can be detected with a sensitivity of 89% and a specificity of 96%.

### 2. Method for Diagnosing Cancer of Digestive Organ

The invention also provides a method for diagnosing a cancer of a digestive organ using, as an indicator, an amount (concentration) of cysteine persulfide or homocysteine persulfide in a sample derived from a subject.

The diagnostic method of the invention is characterized in that the amount (concentration) of cysteine persulfide or homocysteine persulfide in the sample derived from the subject is measured, and when the amount (concentration) of cysteine persulfide or homocysteine persulfide is equal to or greater than a predetermined cutoff value, it is diagnosed that there is a high possibility that the subject has the cancer of the digestive organ.

In the diagnostic method of the invention, the measurement of the amount (concentration) of cysteine persulfide or homocysteine persulfide, the sample derived from the subject, and the cancer of the digestive organ are as described above.

### 3. Method for Treating Cancer of Digestive Organ (Diagnosis + Treatment Method)

The invention also provides a method for treating a cancer of a digestive organ, the method including diagnosing whether a subject has the cancer of the digestive organ by the diagnosis method described above and performing appropriate treatment on the subject diagnosed as having the cancer of the digestive organ.

The method for treating the cancer of the digestive organ is appropriately determined according to a type of the cancer of the digestive organ and a clinical stage thereof. For example, in the case of esophageal cancer, main histological types are squamous cell carcinoma and adenocarcinoma, and respective treatment methods thereof are as follows.

Anti-cancer agents such as i) cisplatin, ii) fluorouracil, iii) docetaxel, and iv) paclitaxel are usually used for treating esophageal squamous cell carcinoma. Agents generally recommended for preoperative chemotherapy are docetaxel + cisplatin + fluorouracil, and cisplatin + fluorouracil is recommended for those such as the elderly where the triple-agent combination is not feasible. For esophageal squamous cell carcinoma for which curative treatment is not possible, an immune checkpoint inhibitor such as pembrolizumab, nivolumab, or ipilimumab may be used in combination in addition to the above agents. Depending on the case, the immune checkpoint inhibitor may be used for the preoperative chemotherapy, or therapy of a combination of fluorouracil + levofolinate + oxaliplatin (FOLFOX) may be used.

For esophageal adenocarcinoma, i) oxaliplatin + S-1 (SOX therapy), ii) docetaxel + oxaliplatin + 5-FU + leucovorin (FLOT therapy), iii) FOLFOX therapy and the like are used as preoperative chemotherapy. In the case of HER2-positive esophageal adenocarcinoma, trastuzumab may be used in combination.

In Europe and the United States, combination therapy of carboplatin + paclitaxel + radiation therapy is generally used for esophageal cancer or esophagogastric junction cancer (both including squamous cell carcinoma and adenocarcinoma) (N Engl J Med. 2012 May 31; 366(22): 2074-84). In addition, a combination of surgical treatment and endoscopic treatment is also useful.

In the treatment method (diagnosis + treatment method) of the invention, the measurement of the amount (concentration) of cysteine persulfide or homocysteine persulfide, the sample derived from the subject, and the cancer of the digestive organ are as described above.

### Examples

### Example 1:

### Target population:

At Tohoku University Hospital, exhaled breath condensate (EBC) and plasma samples were collected from a total of 54 patients diagnosed as having esophageal cancer in a period from July, 2021 to June, 2022 at the time of first visit or hospitalization. EBC sampling was performed before chemotherapy, radiation therapy, dental treatment, and endoscopic treatment were performed, and no restrictions were imposed on diet, smoking, alcohol consumption, or exercise.

As controls, a total of 29 males and females aged 50 to 70 years with no history of cancer were recruited, and EBC and plasma samples were similarly collected.

### Method:

### <EBC Sampling>

A mouthpiece type exhaled breath collector was attached to an exhaled breath condensate apparatus equipped with a Peltier cooling unit, and EBC was collected by rapidly cooling exhaled breath at -20°C. A subject was seated, wore a nose clip, and performed normal breathing through the mouthpiece for 5 minutes. The collected EBC was promptly stored in a freezer at -80°C.

### <Plasma Sampling>

Blood collection was performed at the time of first visit or hospitalization. Whole blood was centrifuged at 3,500 rpm at 4°C for 10 minutes, and a supernatant was stored in a freezer at -80°C.

### <LC-MS/MS Analysis>

### 1. Apparatus and Material

HPLC (Nexera UHPLC, Shimadzu), triple quadrupole mass spectrometer (LCMS-8060NX, Shimadzu), β-4-hydroxyphenyl ethyl iodoacetamide (HPE-IAM), stable isotope-labeled HPE-IAM adduct internal standard (CysSH, CysSSH, CysSSSH, GSH, GSSH, GSSSH, HomoCysSH, HomoCysSSH, HS⁻, HSS⁻, HSSS⁻, HSO₃⁻, HS₂O₃⁻), dimethyl sulfoxide (DMSO)

### 2. EBC Pretreatment

EBC (50 µl) and 4.0 µl of 68 mM HPE-IAM/DMSO are reacted at 37°C for 20 minutes. An alkylation reaction is quenched by adding 1% formic acid. A stable isotope-labeled internal standard with a known concentration is added, and 50 µl is injected into LC-MS/MS.

### 3. Plasma Pretreatment

Plasma (20 µl) and 40 µl of a 5 mM HPE-IAM/90% methanol/50 mM sodium acetate solution are reacted at 37°C for 20 minutes. The mixture is centrifuged at 14,000 rpm at 4°C for 10 minutes, and a supernatant is collected. Formic acid (0.1%) is added to 5 µl of the supernatant to stop an alkylation reaction. A stable isotope-labeled internal standard with a known concentration is added, and 10 µl is injected into LC-MS/MS.

### 4. LC-MS/MS Setting

Column: YMC-Triart C18 (2.0 × 50 mm)
Flow rate: 0.2 mL/min
Mobile phase A: 0.1% formic acid/water, mobile phase B: 0.1% formic acid/methanol
Gradient: liner-gradient (3-95% B: 12 minutes)
Column temperature: 40°C

### <Statistical Processing>

As statistical software, GraphPad Prism version 9 is used. Comparison between two groups was analyzed by Student's t-test, and comparison among three or more groups was analyzed by one-way ANOVA with Tukey's test.

### Results:

In the plasma samples, significant differences were observed between the healthy subjects and the esophageal cancer patients in HS-, CysSH, CysSSH (cysteine persulfide), HomoCysSSH (homocysteine persulfide), and HS₂O₃⁻ (FIG. 2). Meanwhile, in the exhaled breath condensate (EBC) samples, significant differences were observed between the healthy subjects and the esophageal cancer patients in HSS⁻, CysSH, CysSSH, and HS₂O₃⁻ (FIG. 3). In particular, CysSSH was significantly detected at a high concentration in the esophageal cancer patients in both plasma and EBC. In addition, although HomoCysSSH was a detection limit or below in EBC, HomoCysSSH in plasma exhibited a larger difference between the patients and the healthy subjects as compared to CysSSH.

Performance for detecting esophageal cancer patients with respect to CysSSH in EBC, CysSSH in plasma, and HomoCysSSH in plasma was evaluated using an ROC curve, CysSSH (EBC) had AUC = 0.71 (sensitivity 60% and specificity 96% at a cutoff of 0.37 nM), CysSSH (plasma) had AUC = 0.73 (sensitivity 49% and specificity 96% at a cutoff of 336 nm), and HomoCysSSH had AUC = 0.93 (sensitivity 89% and specificity 96% at a cutoff of 10.8 nM), which were considered to be useful for specifically detecting esophageal cancer patients (FIG. 4).

In order to evaluate whether there is a difference according to a histological type (squamous cell carcinoma: SCC, adenocarcinoma: Adeno) or a clinical stage (stage I-II or III-IV) of esophageal cancer, the esophageal cancer patients were classified into groups and evaluated, and CysSSH in plasma and HomoCysSSH in plasma were higher than those of the healthy subjects in all groups of esophageal cancer patients regardless of the histological type and the clinical stage (FIGS. 5 and 6). In particular, HomoCysSSH in plasma was statistically significantly increased (p < 0.001) even in early-stage esophageal cancer at the clinical stage I-II.

A correlation among the three reactive sulfur species, that is, CysSSH in EBC, HomoCysSSH in plasma, and CysSSH in plasma was evaluated. When absolute values of quantitative values of the plasma samples and the EBC samples were compared, the plasma samples had a higher concentration and a more stable result, and a positive correlation was observed between CysSSH derived from plasma and CysSSH derived from EBC (FIG. 7). Similarly, a correlation was observed between CysSSH and HomoCysSSH derived from plasma (FIG. 8).

### Conclusion:

CysSSH and HomoCysSSH were confirmed to be useful as new biomarkers for esophageal cancer. These markers were useful for distinguishing between a healthy subject and a cancer patient regardless of the type and the stage of progression of esophageal cancer. By detecting CysSSH using EBC, it is possible to detect esophageal cancer at an early stage in a non-invasive manner, and accordingly, it is highly possible to identify an esophageal cancer patient more efficiently.

### Industrial Applicability

The invention is useful for early and non-invasive diagnosis of a cancer of a digestive organ.

All publications, patents, and patent applications cited in the description are herein incorporated by reference in their entirety.

## Claims

1. A method for detecting a cancer of a digestive organ in a subject, the method comprising:
a step of measuring an amount of cysteine persulfide or homocysteine persulfide in a sample derived from the subject, wherein
the sample is a sample derived from the subject other than a digestive organ tissue suspected of having a cancer.

2. The method according to claim 1, wherein
the sample derived from the subject is a sample obtained from exhaled breath or blood of the subject.

3. The method according to claim 1, wherein
the sample derived from the subject is an exhaled breath condensate or plasma of the subject.

4. The method according to claim 1, wherein
the cancer of the digestive organ is squamous cell carcinoma or adenocarcinoma.

5. The method according to any one of claims 1 to 4, wherein
the cancer of the digestive organ is esophageal cancer, colorectal cancer, or pancreatic cancer.

6. The method according to claim 1, wherein
the amount of the cysteine persulfide or the homocysteine persulfide being equal to or greater than a predetermined cutoff value indicates a possibility that the subject has the cancer of the digestive organ.

7. The method according to claim 1, wherein
the amount of the cysteine persulfide or the homocysteine persulfide is measured using a mass spectrometer.

8. The method according to claim 1, wherein
the amount of the cysteine persulfide or the homocysteine persulfide is measured by a stable isotope dilution method.

9. The method according to claim 1, further comprising:
a step of reacting an electrophilic alkylating agent having a hydroxyphenyl group with the sample derived from the subject.

10. The method according to claim 9, wherein
the electrophilic alkylating agent having the hydroxyphenyl group is β-(4-hydroxyphenyl)ethyl iodoacetamide (HPE-IAM).

11. The method according to claim 10, wherein
the HPE-IAM is labeled with a stable isotope.
